(19) Europäisches Patentamt
European Patent Office
Office européen des brevets

(11) Numéro de publication: **0 300 867**
**A2**

(12) # DEMANDE DE BREVET EUROPEEN

(21) Numéro de dépôt: **88401758.3**

(22) Date de dépôt: **06.07.88**

(51) Int. Cl.4: **C 12 M 1/40**
C 12 N 9/08, C 12 M 1/00

(30) Priorité: **10.07.87 FR 8709786**

(43) Date de publication de la demande:
**25.01.89 Bulletin 89/04**

(84) Etats contractants désignés:
**AT BE CH DE ES GB GR IT LI LU NL SE**

(71) Demandeur: **SOCIETE NATIONALE ELF AQUITAINE**
**Société anonyme dite**
**Tour Elf 2, Place de la Coupole La Défense 6**
**F-92400 Courbevoie (FR)**

(72) Inventeur: **Bono, Jean-Jacques**
**74, Chemin du Barrail**
**F-32000 Auch (FR)**

**Chavant, Louis**
**13 rue des Potiers**
**F-31000 Toulouse (FR)**

**Seris, Jean-Louis**
**Chemin Vignats**
**F-64110 Jurancon (FR)**

(74) Mandataire: **Kohn, Armand**
**5 Avenue Foch**
**F-92380 Garches (FR)**

(54) **Procédé et dispositif perfectionnés pour la culture de microorganismes, et leur application, notamment à la production de ligninase.**

(57) Procédé de culture de microorganismes dans un milieu nutritif,liquide ; on plonge dans ce milieu un ou plusieurs supports pour le mycélium, la surface de ces supports présentant une multitude de creux et reliefs se succédant près les uns des autres.

Dispositif pour la réalisation d'une telle culture,qui est équipé avec des supports en question. Application à la production de ligninase.

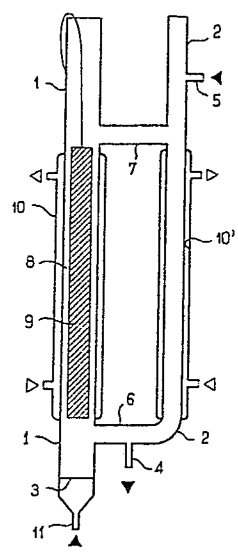


EP 0 300 867 A2

## Description

**Procédé et dispositif perfectionnés pour la culture de microorganismes, et leur application, notamment à la production de ligninase.**

L'invention se rapporte à un perfectionnement dans la culture de microorganismes, plus particulièrement celle de champignons. Elle comprend un dispositif pour la réalisation de ce procédé amélioré, ainsi que des applications de celui-ci. L'invention vise notamment l'application du perfectionnement concerné à la production d'enzymes par des champignons, en particulier de la ligninase.

La biotechnologie a de plus en plus recours à des cultures de microorganismes en vue de la production de différentes substances utiles ; c'est notamment le cas de la préparation de diverses enzymes et d'antibiotiques, dont on connaît l'importance. Etant donné la lenteur des productions biologiques par rapport aux fabrications chimiques, et leur faible rendement par unité de volume de l'appareillage utilisé, des efforts constants sont faits en vue de l'augmentation de ces facteurs. C'est ainsi que le mode opératoire classique des cultures et fermentations, en phase liquide dans des récipients fixes, a été amélioré par l'introduction d'appareils à agitation, à colonnes ou tubulures, à lit fluidisé par un courant d'air, à disques tournants, et autres dispositifs. Cependant, le problème reste toujours posé, puisqu'une réalisation industrielle d'une opération demande une vitesse et un rendement aussi grands que possible. Il se pose particulièrement lorsqu'on travaille sur des champignons ne sécrétant pas de substances polymères et - par conséquent - ne pouvant pas se fixer sur une paroi exposée au renouvellement rationnel de l'oxygène et de liquides appropriés. Tel est notamment le cas de divers basidiomycètes et en particulier du Phanerochaete chrysosporium intéressant à cause de la ligninase qu'il sécrète, mais difficile à cultiver.

La présente invention apporte dans ce domaine une contribution importante : elle permet d'accélérer sensiblement la croissance des champignons qui ne sécrètent pas de matière polymère, d'augmenter la longévité de leurs cultures et d'intensifier la production des substances intéressantes par ces champignons. Ces avantages sont dus à ce que l'invention rend possible le maintien des champignons, ne sécrétant pas de polymères, bien accrochés sur un support solide, pendant que l'on fait passer sur celui-ci un abondant débit d'air ou d'oxygène.

Le procédé de culture suivant l'invention, qui consiste à maintenir le mycélium du champignon sur un support solide, au sein d'un milieu nutritif, en faisant passer de l'air ou de l'oxygène au contact du mycélium, est caractérisé en ce que la surface du support présente une multitude de creux et de reliefs successifs, la distance entre deux reliefs adjacents étant de 0,05 à 0,5 mm et, de préférence, 0,1 à 0,3 mm.

L'effet surprenant de l'invention réside en ce que, malgré l'absence de toute sécrétion de matière polymère, notamment de polysaccharide, qui permettrait au champignon de coller à un support solide, on arrive tout de même à obtenir une fixation ferme du mycélium, lorsque le support comporte les rugosités des dimensions spécifiques, susindiquées. De préférence, la profondeur des creux est de l'ordre de 0,01 à 0,5 mm, de préférence 0,025 à 0,5mm.

Le procédé de culture de microorganismes suivant l'invention consiste ainsi à laisser croître les microorganismes dans un milieu nutritif, liquide, approprié, dans lequel on a disposé un ou plusieurs supports dont la surface présente une multitude de creux et reliefs se succédant les uns aux autres aux distances indiquées plus haut.

Lorsque le mycélium a formé, sur son support, une certaine épaisseur, le support est retiré et remplacé par un autre, non encore recouvert de culture ; s'il y a lieu, le milieu nutritif est alors renouvelé ; il peut d'ailleurs être introduit en continu, dans l'espace de culture, avant et après le retrait du support initial. Dans une forme particulière d'éxécution du procédé de l'invention, lorsque l'épaisseur du mycélium a atteint une certaine valeur, on remplace le milieu liquide par un autre, prévu pour faire sécréter aux microorganismes les substances désirées.

Le nouveau procédé s'applique tant au travail aérobie qu'aux cultures anaérobies. Dans le premier cas, il peut être avantageusement réalisé avec les méthodes connues de soufflage de l'air ou de l'oxygène dispersé, ou la flottation.

Le procédé suivant l'invention est mis en pratique dans un appareil comportant une chambre à culture, statique ou munie de moyens de circulation de liquide et - éventuellement - de gaz, caractérisé en ce que, dans cette chambre, sont placés un ou plusieurs supports définis plus haut.

Lorsque la chambre à culture est à circulation de milieu liquide, le ou les supports sont suspendus de préférence longitudinalement suivant le sens de circulation du liquide.

Selon une forme préférée de l'invention, le support, offert au mycélium, est constitué par un tissu, un gril lage ou un écheveau, dont les fils ou rubans constitutifs peuvent être en des matières très diverses, à condition de ne pas être attaquables par les microorganismes ou répulsifs vis-à-vis de ceux-ci. Ainsi, le support peut être en métal, en fibre de verre ou autre minéral, ou bien en l'une des nombreuses matières polymères connues à l'heure actuelle. Des exemples non limitatifs sont des grilles en acier inoxydable, tissu de fibre de verre, gaze ou trame d'acétate de cellulose, de polyester, polyacrylonitrile, polyamide (Nylons), polyéthylène, polyacrylate, etc.

Une variante comprend, en tant que supports de mycélium, des plaques ou feuilles dont la surface est sculptée, gaufrée ou rainurée en fins reliefs et creux, très rapprochés les uns des autres. Les mêmes matières, indiquées plus haut, conviennent. En particulier peut-on employer des plaques gaufrées

en poly(chlorure de vinyle), polystyrène, phénoplaste,polypropylène, polyuréthanne, etc.

Comme l'écart entre deux reliefs ou fils, dans le support de mycélium suivant l'invention est de 0,05 à 0,5 mm, ou mieux, de 0,1 à 0,3 mm. Avec des grilles, trames et tissus, d'excellents résultats ont été obtenus pour des écarts d'environ 0,1 à 0,3 mm entre deux fils adjacents, ce qui correspond à des tamis N°21 à 29 selon les normes AFNOR (tamis TYLER standard 60 à 150).

Les dimensions des supports dépendent, bien entendu, de celles de l'appareil utilisé pour la culture donnée, mais il est en général préférable que la longueur du support soit 5 à 25 fois la largeur : cela permet de disposer les supports lonqitudinalement dans le sens de la circulation du liquide dans l'appareil.

Le principe de la présente invention peut s'appliquer aux dispositifs connus pour la culture de microorganismes : il suffit pour cela de placer un ou plusieurs supports suivant l'invention dans la chambre de culture de l'appareil. Une forme d'exécution particulièrement intéressante est celle où un appareil, du type à soufflage de l'air dispersé et à circulation de liquide, est muni d'un ou de plusieurs supports selon l'invention dans la tubulure servant de chambre de culture.

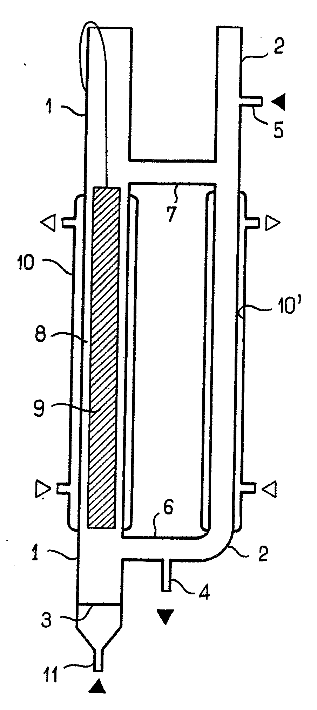

A titre d'exemple non limitatif de nouveau dispositif, on en décrit un ci-aprés, avec référence au dessin annexé.

L'appareil comprend deux tubulures, 1 et 2, communiquant au bas, 6, et en haut, 7, d'une partie 8 servant de chambre de culture. Une canalisation latérale 5 permet l'approvisionnement en le milieu de culture, dont le soutirage se fait en bas, par 4. Au bas de la tubulure 1 sont placés des moyens de dispersion 3 du gaz sous pression introduit par 11 ; ces moyens sont constitués par exemple par une plaquette de verre fritté ou par une fine grille métallique. Les deux branches, 1 et 2, sont entourées d'enveloppes 10 et 10', pour le passage d'un fluide calorifique, permettant de régler la température à la valeur voulue à l'intérieur de l'appareil.

La caractéristique importante, suivant l'invention, est la présence d'un support 9 placé longitudinalement dans la tubulure 1. Ce support peut avoir les forme et nature décrites plus haut. Dans des cas particuliers, de très bons résultats ont été obtenus avec des supports constitués par de la trame de polyamide 6 ou 6.6 (Nylon 6 ou 6.6).

La pose amovible du support 9 dans la chambre 1 peut s'effectuer par tous moyens connus, notamment par suspension, ou par calage sur le fond de la tubulure 1 ou sur des pièces prévues à cet effet, non représentées sur le dessin.

Le procédé et l'appareil suivant l'invention peuvent s'appliquer à diverses cultures bactériennes ou fongiques, et tout particulièrement à celles de certains champignons. Ils sont illustrés non limitativement par l'application à la culture du champignon basidomycète Phanerochaete Chrysosporium et la production de l'enzyme ligninase par ce microorganisme.

Les ligninases du champignon basidiomycète Phanerochaete chrysosporium Burds sont des peroxydases extracellulaires, capables de cliver des liaisons caractéristiques de lignines. Aussi leur utilisation potentielle comme agent de délignification, dans des domaines tels que l'alimentation des ruminants ou le blanchiment des pâtes à papier, a-t-elle été envisagée. Cependant, outre les difficultés que présente une mise en oeuvre de ces enzymes dans un procédé biotechnologique, leur production, dans des conditions transposables à grande échelle, n'a pas encore été réalisée.

Les ligninases ont été décrites comme des enzymes sécrétées dans le milieu de culture de Phanerochaete chrysosporium, lorsque celui-ci est cultivé en milieu synthétique dans des fioles d'erlenmeyer non agitées (TIEN et KIRK 1983, Science, 221, 661-663). Par la suite, d'autres modes de culture, destinés à accroître la productivité de ligninases, ont été proposés : en milieu agité (RENGANATHAN et coll. Arch. Biochem. Bio-Phys. 241,304-312 ; JAGER et coll. Appl. Environ. Microbiol. 1985, 50, 1274-1278 ; LEISOLA et FIECHTER, 1985, FEMS. Microbiol. Lett. 29,33-36), avec un dispositif rotatif, (KIRK et coll. 1986 Enzyme Microb. Technol. 8, 27-32 ; PASZCZYINSKI et coll. 1986, FEMS Microb. lett. 29, 37-41); sous forme de mycélium immobilisé (LINKO et coll. 1986 Journ. Bio-Techn. 4, 283-291). Aucun de ces travaux n'a conduit à une production industrielle. L'application de la présente invention, par contre, permet une production de ligninases compatible avec une expérimentation à grande échelle.

Dans les conditions dés exemples qui suivent, le champignon est d'abord fixé sur une trame de nylon. Ensuite, la trame est introduite dans un réacteur, comme celui du dessin annexé, contenant le milieu de culture. Un courant d'air ou d'oxygène sous pression est utilisé pour homogénéiser le milieu et établir des conditions favorables à la croissance fongique, et à l'expression du système ligninolytique. Lorsque la production des ligninases atteint son maximum, le milieu de culture est récupéré dans sa totalité. Le réacteur est alors alimenté en milieu neuf, qui permet ultérieurement l'obtention d'un nouveau lot de jus actif. Ce processus peut être répété durant plusieurs semaines. Le mode opératoire comportait les opérations que voici :

1. Fixation du champignon

Le champignon est mis en culture dans une fiole d'erlenmeyer de 1 litre, contenant 400 ml de milieu nutritif et une trame de nylon de 30 x 2 cm avec 0,25 mm de vide de maille. Le milieu avait la composition en g/l :

$KPH_2PO_4$ 2
$Mg\ SO_4\ 7H_2O$ 0,5
$Ca\ Cl_2\ 2H_2O$ 0,1
$(NH_4)_2\ C_4H_4O_6$ 2,4
Thiamine HCl $0{,}2x10^{-3}$
Glycérol 5
Tween 80 1
Alcool vératrylique (activateur) $67x10^{-3}$
Solution minérale 7 ml

Ce milieu est tamponné à pH 4,5 par de l'acétate de Na 10 mM. La solution minérale, employée, avait la composition en g/l :

$Mg\ SO_4\ 7H_2O$ 3
$Mn\ SO_4\ H_2O$ 0,5
Na Cl 1
$Fe\ SO_4\ 7H_2O$ 0,1
$Co\ Cl_2$ 0,1
$Zn\ SO_4\ 7H_2O$ 0,1
$Cu\ SO_4\ 5H_2O$ 0,01
$Ca\ Cl_2\ 2H_2O$ 0,1
$Al\ K\ (SO_4)_2\ 12H_2O$ 0,01
$H_3\ BO_4$ 0,01
$Na\ MoO_4\ 2H_2O$ 0,01

L'ensemencement était réalisé par des spores en suspension dans une solution aqueuse de "Tween 80" à 0,1%, à raison de 0,25 $x10^6$ spores/ml de milieu. La culture s'est poursuivie 24 heures à 35°C sous agitation.

Dans ces conditions, le mycélium s'est présenté sur la forme de paillettes fixées sur la trame.

2. Croissance en réacteur à soufflage d'air dispersé

Le réacteur. constitué par les deux compartiments, 1 et 2, en verre, reliés entre eux, selon dessin annexé, avait une capacité de 1 litre.

La chambre 8 du compartiment 1 reçoit la trame 9 ; par la plaque de verre fritté 3 on réalisait une bonne diffusion de l'air ou d'$O_2$ dans le milieu de culture.

Le compartiment 2 permettait une circulation du milieu dont la température optimale de croissance du champignon était réglée à 35°C.

Après introduction de la trame dans le réacteur, on faisait passer en continu des bulles d'air, durant une période de 48 heures, avec un débit de 100 ml/mn, nécessaire au développement du mycélium. A partir de là, la culture fut maintenue sous $O_2$ pur tout au long de l'expérimentation.

EXEMPLE 1

On a maintenu le champignon en culture pendant plus de 2 mois dans les conditions décrites plus haut. L'activité ligninase était suivie au cours de ce temps de la façon suivante.

20 ml de milieu sont prélevés ; le volume de la culture est maintenu constant par l'apport du tampon acétate 10 mM pH 4,5. L'activité enzymatique est déterminée par la méthode de TIEN et KIRK (1983), exposée dans la publication citée plus haut, basée sur l'oxydation de l'alcool vératrylique

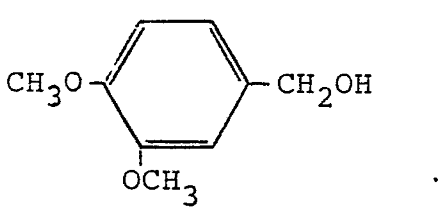

en l'aldéhyde correspondant par de l'eau oxygénée. On opérait sur 3 ml de mélange réactionnel composé de : 1ml de jus de culture, de tampon glycine-HCl, 0,1 MpH3, 0,4 mM d'alcool vératrylique et 0,1 mM de $H_2O_2$. L'unité de l'activité de la ligninase est définie comme la quantité de cette enzyme qui conduit à la formation de 1 micromole d'aldéhyde vératrylique par minute.

L'activité du jus de la culture est de 230 U/l soit plus de 1,5 fois le maximum atteint par Alexander JAGER et coll. (Applied and Environmental Microbiology, Nov. 1985-vol. 50, n°5, page 1276, Tableau 2).

Lorsque la production de ligninase atteint son maximum, la totalité de jus de culture est récupérée. Le milieu de remplacement est identique au milieu de base, mais les concentrations des différents constituants sont divisées par 10 à l'exception de celles du tampon, de l'alcool vératrylique et du "Tween 80".

ESSAI COMPARATIF

Les opérations de l'exemple 1 étant répétées sans le support de nylon dans le réacteur, la production de ligninase est très faible.

EXEMPLE 2

Les opérations de l'exemple ont été répétées, mais le tampon d'acétate de Na, pH 4,5 fut remplacé par un tampon de diméthyl-2,2 succinate de Na de même concentration 10mM, pH également 4,5.

Les résultats sont alors moins bons, mais encore meilleurs que sans support de nylon.

EXEMPLE 3

On procède comme dans l'exemple 1, mais l'appareil fonctionne en continu à raison de 15 ml de milieu de remplacement introduit par heure ; le même débit d'oxygène de 100 ml/h que dans l'exemple 1 est maintenu en permanence. On arrive ainsi à une production de ligninase d'activité moyenne constante de 100 U/l, qui a été maintenue pendant 5 jours.

**Revendications**

1. Procédé de culture de microorganismes dans un milieu nutritif,liquide, où l'on plonge un ou plusieurs supports pour le mycélium, caractérisé en ce que la surface de ces supports présente une multitude de creux et reliefs, la distance entre deux reliefs adjacents étant de 0,05 à 0,5 mm.

2. Procédé suivant la revendication 1, caractérisé en ce que le ou les supports sont retirés, lorsque le mycélium sur leur surface a atteint une certaine grandeur, pour être éventuellement remplacés par de nouveaux supports.

3. Procédé suivant la revendication 1 ou 2, caractérisé en ce que la profondeur des creux est de l'ordre de 0,01 à 0,5 mm et de préférence de 0,025 à 0,5 mm.

4. Procédé suivant une des revendications 1 à 3, caractérisé en ce que le support est

constitué par un tissu, grillage, écheveau ou bien feuille ou plaque à surface gaufrée ou rainurée, en métal, verre ou autre minéral, ou polymère, en particulier textile.

5. Procédé suivant une des revendications 1 à 4, caractérisé en ce que les écarts préférés entre deux reliefs successifs dans le support sont de 0,1 à 0,4 mm.

6. Dispositif pour la culture de microorganismes, qui comporte une chambre de culture et des moyens pour l'introduction et soutirage de liquide (4,5) et des moyens pour le soufflage de gaz (3,11), caractérisé en ce qu'un ou plusieurs supports (9) sont placés dans cette chambre (8), de préférence longitudinalement par rapport au passage de liquide, ces supports présentant une surface avec une multitude de creux et de reliefs, la distance entre deux reliefs adjacents étant de 0,05 à 0,5 mm et de préférence 0,1 à 0,3 mm.

7. Dispositif suivant la revendication 6, caractérisé en ce que le support (9) est un tissu, grille, écheveau ou plaque gaufrée, en métal, verre ou autre minéral, ou polymère, en particulier textile.

8. Dispositif suivant la revendication 6 ou 7, caractérisé en ce que la profondeur des creux est de 0,01 à 0,5 mm et de préférence de 0,025 à 0,5 mm.

9. Application du procédé suivant une des revendications 1 à 5 à la culture du champignon Phanerochaete chrysosporium en vue de la production de la ligninase.

10. Application suivant la revendication 9, caractérisée en ce que le support utilisé est une trame de polyamide placée dans la chambre de culture longitudinalement par rapport au passage du milieu nutritif et de l'oxygène.

11. Application suivant la revendication 9 ou 10, caractérisée en ce que la culture a lieu dans un milieu tamponné à l'acétate de sodium à pH 4,5.

0300867